# DEMANDE DE BREVET EUROPEEN

(11) **EP 2 368 595 A1**
(43) Date de publication de la demande: **28.09.2011**
(21) Numéro de dépôt: 10157708.8
(22) Date de dépôt: 25.03.2010
(51) Int. Cl.: A61M 25/02

(54) **Dispositif de guidage de tuyau(x) de perfusion**

(71) Demandeur: Arn, Julien, 2340 Le Noirmont (CH)
(72) Inventeur: Arn, Julien, 2340 Le Noirmont (CH)
(74) Mandataire: P&TS Patents & Technology Surveys SA

(57) **Abrégé**

La présente invention concerne un dispositif de guidage de tuyau(x) de perfusion (10), comportant un bracelet (11) muni d'un organe de fermeture (14) pour permettre son attache à un membre d'un patient et portant une plaquette de guidage (12) d'au moins un tuyau de perfusion (5, 6). Le dispositif est caractérisé par le fait que la plaquette de guidage (12) présente, ménagées dans son épaisseur, au moins une première gorge de guidage (16) d'un tuyau (5), sensiblement en forme de U, et une seconde gorge de guidage (17) sensiblement rectiligne, suivant la direction longitudinale du bracelet (11), et agencée tangentiellement à la première gorge de guidage (16), de telle manière qu'un tuyau (5) peut être disposé en partie dans une première des gorges de guidage et en partie dans l'autre gorge de guidage.

## Description

### Domaine technique

La présente invention concerne un dispositif de guidage de tuyau(x) de perfusion, comportant un bracelet muni d'un organe de fermeture pour permettre son attache à un membre d'un patient et portant une plaquette de guidage d'au moins un tuyau de perfusion.

Un tel dispositif est destiné à améliorer le confort d'un patient devant notamment subir une perfusion de manière prolongée, ainsi que sa sécurité, du fait qu'il permet de limiter le risque d'arrachement accidentel de la perfusion.

La présente invention concerne également un procédé de fabrication d'un tel dispositif.

### Etat de la technique

De nombreux dispositifs de ce type ont été divulgués dans l'état de la technique, comme par exemple dans le brevet US 5,916,199 du 29 juin 1999. Ce document décrit un dispositif de guidage d'un tuyau de perfusion comportant un bracelet de type à fermeture par système Velcro (marque déposée) sur lequel est enfilée une plaquette de guidage. Cette dernière comprend une base et un couvercle relié à la base de manière à pouvoir pivoter entre des positions ouverte et fermée. La base et le couvercle comprennent chacun un canal en forme de U, les canaux étant agencés de manière à coopérer l'un avec l'autre dans la position fermée du couvercle pour définir un logement de guidage pour un tuyau de perfusion.

Cependant, il faut noter que ce dispositif présente un nombre important d'inconvénients. Sa structure est complexe ce qui entraîne non seulement un procédé de fabrication coûteux, mais également une lenteur relative dans sa mise en place sur le membre d'un patient. En effet, la plaquette de guidage doit être enfilée sur le bracelet avant de rendre celui-ci solidaire du membre d'un patient perfusé, suite à quoi le tuyau doit être placé dans le canal de la base avant de procéder à la fermeture de la plaquette par pivotement du couvercle.

En outre, on notera que ce dispositif est dénué d'intérêt lorsque l'aiguille de perfusion est insérée, par exemple, dans la main, dans le sens allant de l'épaule vers la main (soit la configuration inverse de celle illustrée sur la figure 1 de ce brevet). Dans ce cas, seul un dispositif de guidage accessoire également décrit dans ce document, présentant un logement de guidage rectiligne, peut être mis en oeuvre pour assurer le guidage du tuyau vers le haut du corps du patient.

La demande de brevet WO 2009/137607 A1, publiée le 12 novembre 2009, décrit un autre type de dispositif de guidage pour tuyau de perfusion, présentant un mode de mise en place du tuyau sur des organes de guidage simplifié par rapport au dispositif décrit ci-dessus. La structure divulguée pour les organes de guidage du tuyau offre en outre une plus grande souplesse pour adapter le guidage du tuyau au mode d'implantation de la perfusion sur le membre du patient.

Toutefois, le nombre de constituants du dispositif et leurs formes rend le procédé de fabrication et d'assemblage du dispositif complexe et couteux. Il convient de noter que l'exploitation de ces dispositifs relève de la grande série. Chaque hôpital devrait pouvoir s'équiper de tels dispositifs avec une quantité supérieure au nombre de lits qu'il comporte. Par conséquent, le prix d'achat d'un dispositif de ce type pour un hôpital devrait être aussi bas que possible.

Par ailleurs, on notera encore que l'un et l'autre des dispositifs de l'art antérieur décrits ci-dessus présentent un inconvénient supplémentaire, également lié aux structures correspondantes. Pour en rendre l'usage aisé, de tels dispositifs devraient pouvoir être nettoyés et stérilisés facilement et rapidement, entre deux patients. Or, l'un comme l'autre des dispositifs qui viennent d'être décrits présentent notamment des creux ou petites anfractuosités dans lesquels des souillures peuvent se loger facilement, rendant leur nettoyage laborieux.

### Divulgation de l'invention

Un but principal de la présente invention est de pallier les inconvénients des dispositifs de guidage de tuyau de perfusion connus de l'art antérieur, en proposant un tel dispositif d'utilisation simple et flexible.

A cet effet, la présente invention concerne plus particulièrement un dispositif de guidage de tuyau(x) de perfusion du type mentionné plus haut, caractérisé par le fait que la plaquette de guidage présente, ménagées dans son épaisseur, au moins une première gorge de guidage d'un tuyau sensiblement en forme de U et une seconde gorge de guidage sensiblement rectiligne, suivant la direction longitudinale du bracelet, et agencée tangentiellement à la première gorge de guidage, de telle manière qu'un tuyau peut être disposé en partie dans une première des gorges de guidage et en partie dans l'autre gorge de guidage.

Grâce à ces caractéristiques, le dispositif de guidage selon l'invention présente une structure simple impliquant une mise en place du tuyau aisée et flexible, c'est-à-dire susceptible d'être facilement adaptée au mode d'implantation de la perfusion sur le patient.

De manière préférée, la plaquette de guidage comporte au moins une troisième gorge de guidage sensiblement parallèle à la seconde gorge de guidage. Cette caractéristique particulière permet d'effectuer le guidage d'un tuyau supplémentaire, notamment dans le cas d'une perfusion à deux entrées simultanées.

Selon un mode de réalisation préféré, chacune des gorges de guidage présente, en section transversale à partir de la surface libre de la plaquette de guidage, deux parois sensiblement parallèles se rejoignant en une courbe sensiblement en forme de demi-cercle. En outre, chacune des gorges comprend au moins une région présentant au moins une surépaisseur ménagée sur au moins l'une des parois, à proximité de la surface libre de la plaquette de guidage, et définissant un étranglement.

Ainsi, la mise en place d'un tuyau dans une gorge est très simple puisqu'il suffit de l'y insérer par pression, le tuyau subissant alors une légère compression passagère, lors du franchissement de l'étranglement.

De manière particulièrement avantageuse, le bracelet et la plaquette de guidage, voire l'organe de fermeture, sont formés d'une seule pièce.

Grâce à ces caractéristiques, non seulement le procédé de fabrication du dispositif selon l'invention est simplifié en référence aux procédés de fabrication des dispositifs de l'art antérieur, mais en outre, il est possible de contrôler précisément la forme du dispositif, notamment pour éviter la formation d'anfractuosités dans lesquelles des saletés pourraient se loger en cours d'utilisation ultérieure.

De manière similaire, l'organe de fermeture devrait avantageusement présenter la structure la plus simple possible, tant pour des raisons de fabrication que de nettoyage du dispositif. Ainsi, on peut notamment prévoir qu'il comporte un bouton agencé pour coopérer avec un trou parmi une pluralité de trous ménagés dans le bracelet.

Par ailleurs, les caractéristiques particulières du dispositif qui viennent d'être énumérées peuvent être obtenues par la mise en oeuvre d'un procédé de fabrication comportant les étapes consistant à:
a) se munir d'un moule d'injection correspondant à la forme souhaitée pour le bracelet et la plaquette de guidage;
b) se munir d'au moins un tiroir permettant de ménager, dans l'épaisseur de la plaquette de guidage, au moins une première gorge de guidage d'un tuyau sensiblement en forme de U et une seconde gorge de guidage sensiblement rectiligne, suivant la direction longitudinale du bracelet, et agencée tangentiellement à la première gorge de guidage;
c) effectuer un moulage par injection au moyen du moule et du ou des tiroirs pour former le dispositif de guidage.

### Brève description des dessins

D'autres caractéristiques et avantages de la présente invention apparaîtront plus clairement à la lecture de la description détaillée d'un mode de réalisation préféré qui suit, faite en référence aux dessins annexés donnés à titre d'exemples non limitatifs et dans lesquels:

- la figure 1 représente une vue de face simplifiée d'un exemple de mise en oeuvre du dispositif de guidage selon un mode de réalisation préféré de la présente invention, dans une première configuration;

- la figure 2 représente une vue similaire à celle de la figure 1, dans une seconde configuration;

- la figure 3 représente une vue de face simplifiée d'un dispositif de guidage selon un mode de réalisation préféré de la présente invention;

- la figure 4 représente une vue latérale simplifiée du dispositif de guidage de la figure 3, et

- la figure 5 représente une vue de dos simplifiée du dispositif de guidage de la figure 3.

### Mode(s) de réalisation de l'invention

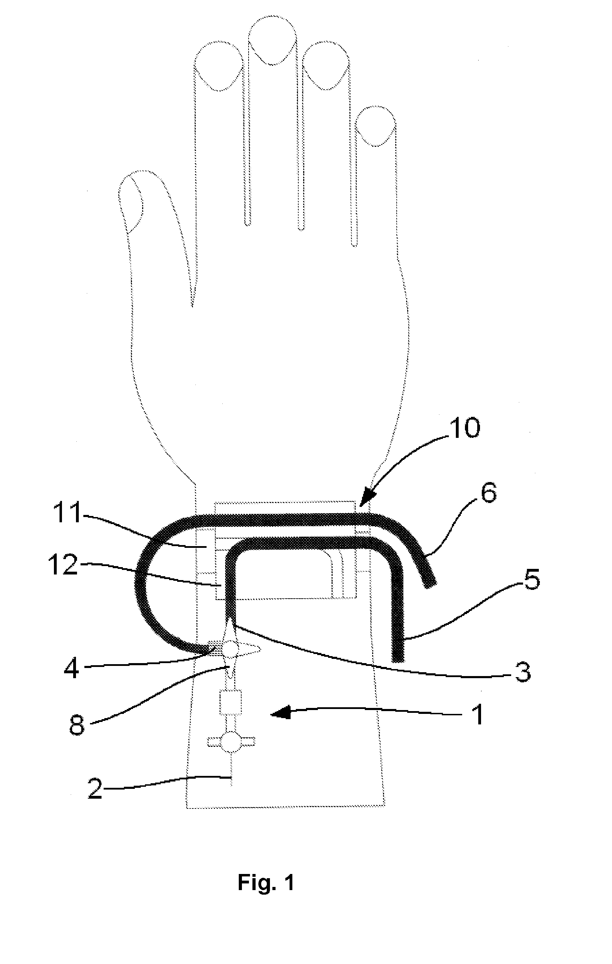

La figure 1 représente une vue de face simplifiée d'un exemple de mise en oeuvre du dispositif de guidage selon un mode de réalisation préféré de la présente invention, dans une première configuration.

Plus précisément, la figure 1 illustre la mise en place d'une perfusion 1 sur l'avant-bras d'un patient. L'aiguille 2 est implantée dans l'avant-bras dans le sens de la main vers l'épaule. De ce fait, l'arrivée du ou des tuyaux d'alimentation en liquide à perfuser est située du côté de la main.

Dans l'exemple représenté ici, la perfusion comporte des première et seconde arrivées 3 et 4, chacune étant reliée à un tuyau 5, 6.

La seconde arrivée 4 est munie d'un robinet 8 et est orientée vers le corps du patient, la première arrivée étant orientée vers la main du patient.

Le patient porte un dispositif de guidage de tuyaux 10 selon un mode de réalisation préféré de la présente invention. Celui-ci comprend un bracelet 11 portant une plaquette de guidage 12 des tuyaux. Il ressort de la figure 1 que les tuyaux 5 et 6 sont mis en place sur la plaquette de guidage 12 et sont guidés pour être orientés vers le haut du corps du patient, par l'extérieur de son bras.

Grâce à ce guidage, et du fait que le maintien des tuyaux sur la plaquette de guidage est sûr, le patient dispose d'une plus grande liberté de mouvement qu'avec certains dispositifs de guidage de tuyaux de l'art antérieur. En particulier, on notera que le dispositif de guidage selon le présent mode de réalisation préféré permet avantageusement d'assurer le guidage simultané de deux tuyaux, ce qui n'était pas le cas des dispositifs de l'art antérieur présentés plus haut.

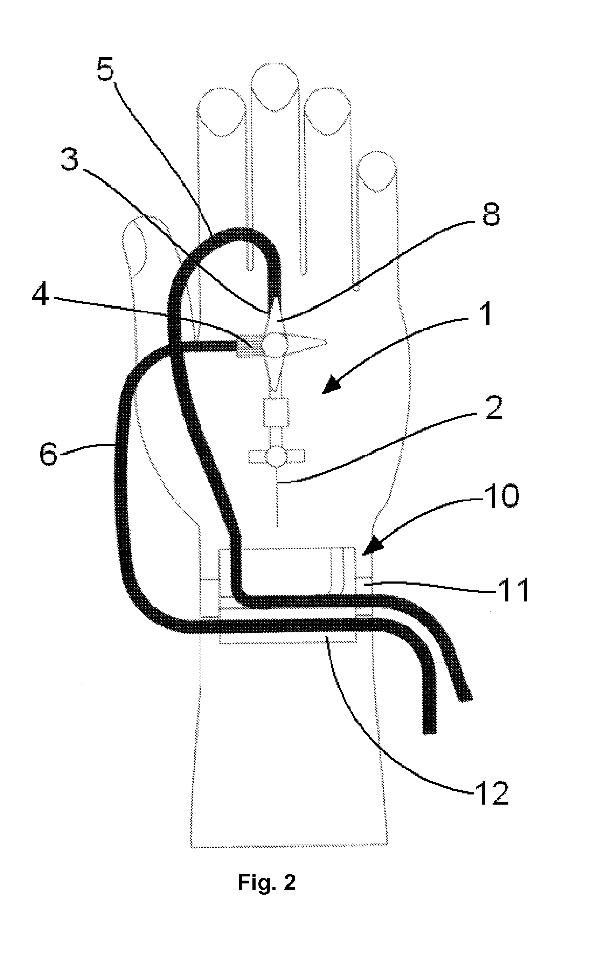

La figure 2 représente une vue similaire à celle de la figure 1, dans une seconde configuration.

Plus précisément, la figure 2 illustre la mise en place de la perfusion 1 directement sur la main du patient. L'aiguille 2 est implantée dans la main dans le sens de la main vers l'épaule. De ce fait, l'arrivée du ou des tuyaux d'alimentation en liquide à perfuser est située du côté de la main, de même que dans le cas de la figure 1.

Toutefois, contrairement à la configuration de la figure 1, les tuyaux 5 et 6 ne s'étendent pas en direction du poignet du patient à partir de la perfusion 1, mais en direction de ses doigts.

Ainsi, de manière avantageuse, les tuyaux 5, 6 peuvent être disposés de façon à revenir en direction du poignet où est agencé le dispositif de guidage selon la présente invention, par le côté de la main situé près du corps du patient. Les tuyaux peuvent alors être mis en place sur la plaquette de guidage 12 de telle manière qu'ils sont ensuite orientés vers le haut du corps du patient, par l'extérieur de son bras, de même que dans le cas de la configuration de la figure 1.

On constate que le dispositif de guidage de tuyaux 10 est disposé en sens inverse dans la configuration de la figure 2, en référence à la configuration de la figure 1, son retournement entre l'une et l'autre configuration permettant d'obtenir le même résultat final avantageux, tel qu'exposé précédemment en relation avec la figure 1.

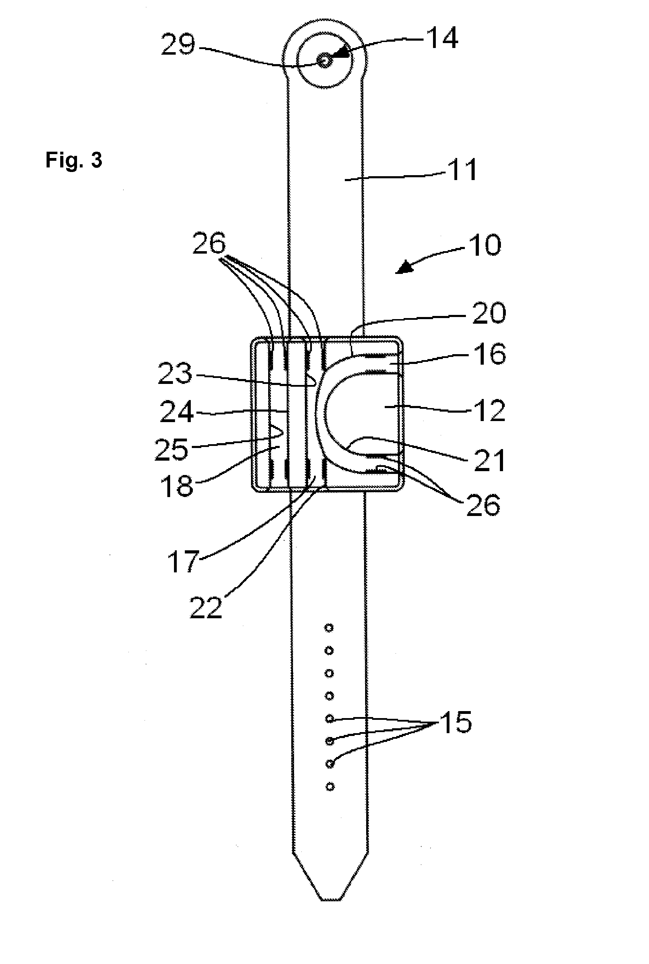

Un dispositif de guidage 10 selon un mode de réalisation préféré de la présente invention va être décrit plus en détail sur la base des figures 3, 4 et 5.

La figure 3 représente une vue de face simplifiée de ce dispositif de guidage 10.

Le bracelet 11 comprend un organe de fermeture 14 réalisé ici sous la forme d'un bouton, situé à une première extrémité du bracelet et, destiné à coopérer avec des trous 15 ménagés à proximité de la seconde extrémité du bracelet, de manière conventionnelle. On notera qu'un tel organe de fermeture présente une grande simplicité tant dans sa fabrication que dans son utilisation.

La plaquette de guidage 12 est agencée dans la région médiane du bracelet 11.

Il ressort clairement de la figure 3 que la plaquette de guidage comporte une première gorge de guidage 16 d'un tuyau, sensiblement en forme de U, ainsi qu'une seconde gorge de guidage 17 sensiblement rectiligne, suivant la direction longitudinale du bracelet, et agencée tangentiellement à la première gorge de guidage, de sorte que les deux gorges communiquent l'une avec l'autre. Grâce à cette dernière caractéristique, un tuyau peut être disposé en partie dans une première des gorges de guidage et, simultanément, en partie dans l'autre gorge de guidage. Une telle disposition ressort des configurations représentées sur les figures 1 et 2, pour le premier tuyau 5.

Par ailleurs, selon le mode de réalisation préféré tel qu'illustré sur les figures, la plaquette de guidage 12 comprend une troisième gorge de guidage 18 sensiblement parallèle à la seconde gorge de guidage 17, dans le but d'assurer le guidage d'un tuyau supplémentaire, comme le tuyau 6 illustré sur les figures 1 et 2.

Les avantages à l'utilisation du dispositif de guidage selon ce mode de réalisation préféré apparaissent nettement des figures 1 à 3, notamment le fait qu'il peut être utilisé dans un sens comme dans l'autre en fonction de la région d'implantation de la perfusion sur le membre d'un patient.

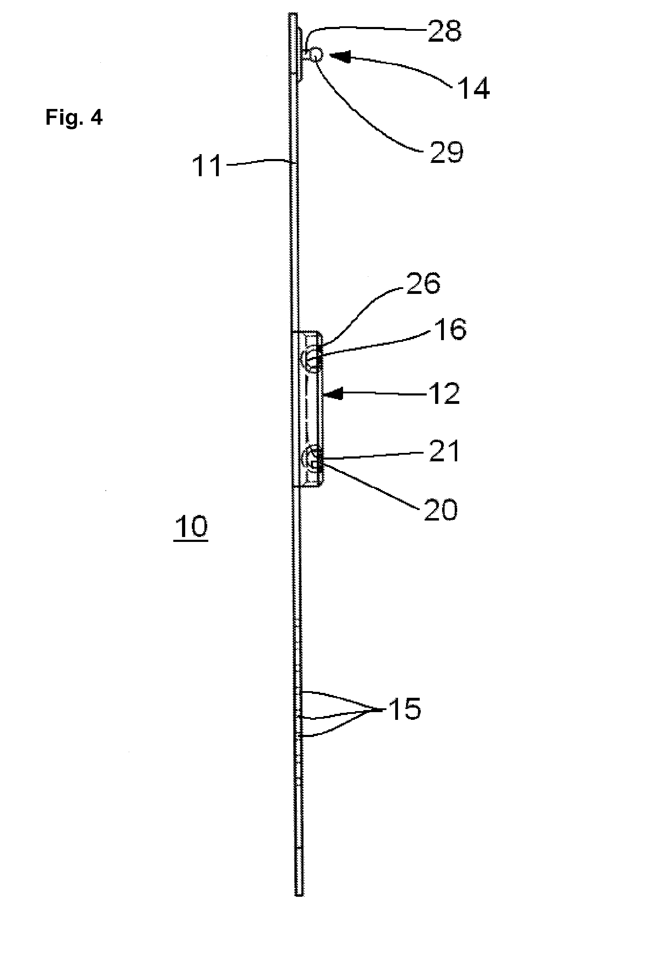

La figure 4 représente une vue latérale simplifiée du dispositif de guidage de la figure 3, de laquelle il ressort, en combinaison avec la figure 3, que chacune des gorges 16, 17 et 18 comprend deux parois 20 et 21, 22 et 23, 24 et 25, sensiblement parallèles et se rejoignant deux-à-deux en une courbe sensiblement en forme de demi-cercle.

Bien entendu, de manière alternative, il est possible de prévoir que les parois sont légèrement incurvées, tel que cela ressort de la figure 4 pour les parois 20 et 21, sans sortir du cadre de la présente invention. Selon cette alternative, la première gorge de guidage 16 présente une section transversale en forme de portion de cercle.

Dans un cas comme dans l'autre, il est préférable de prévoir en outre que chacune des gorges comprend au moins une région présentant au moins une surépaisseur 26 ménagée sur au moins l'une de ses parois, à proximité de la surface libre de la plaquette de guidage 12, et définissant un étranglement. En particulier, selon le présent mode de réalisation préféré, chaque gorge de guidage est munie de quatre surépaisseurs 26 pour garantir un bon maintien en place du tuyau guidé le long de chaque gorge.

Comme mentionné précédemment, la mise en place d'un tuyau dans une gorge est très simple puisqu'il suffit de l'y insérer par pression, le tuyau subissant alors une légère compression passagère, lors du franchissement de l'étranglement.

On notera en outre que le dispositif selon l'invention présente l'avantage supplémentaire de garantir en tout temps une bonne visibilité des tuyaux, ce qui permet de contrôler qu'ils ne sont pas obstrués lors de l'administration d'un produit à un patient.

Par ailleurs, il ressort de la figure 4 que le bouton de fermeture 14 comprend ici une base 28 sensiblement cylindrique surmontée d'une boule 29 destinée à assurer la retenue du bouton dans l'un des trous 15.

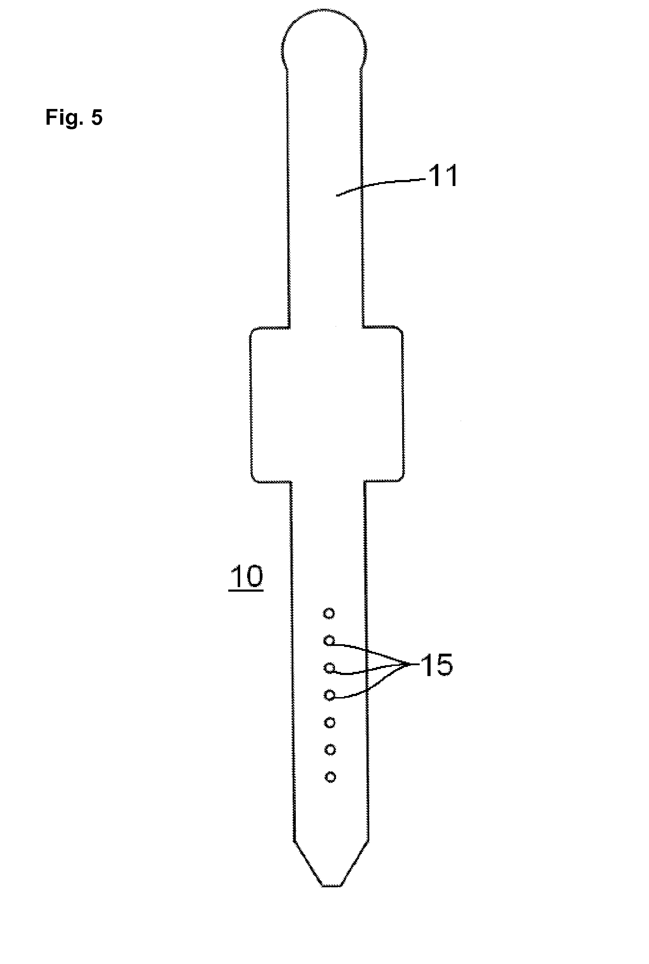

La figure 5, qui représente une vue de dos simplifiée du dispositif de guidage de la figure 3, permet en outre d'apprécier la simplicité structurelle du dispositif de guidage selon le présent mode de réalisation préféré de l'invention.

Il apparaît en effet de la figure 5 que le dos du dispositif est parfaitement lisse et ne présente aucune ouverture, autre que les trous de fermeture 15, qui pourrait donner lieu à une accumulation de saletés et qui pourrait compliquer le nettoyage du dispositif entre deux utilisations successives.

De manière avantageuse, le bracelet 11 peut être réalisé d'une seule pièce en matière plastique, préférablement par moulage. Bien entendu, il est possible d'utiliser plusieurs matières plastiques aux propriétés mécaniques différentes adaptées aux contraintes que doivent subir les différentes parties du dispositif.

Plus précisément, on peut prévoir que le dispositif de guidage selon l'invention est fabriqué par la mise en oeuvre d'un procédé de fabrication comportant les étapes consistant à:
a) se munir d'un moule d'injection correspondant à la forme souhaitée pour le bracelet et la plaquette de guidage;
b) se munir d'au moins un tiroir permettant de ménager, dans l'épaisseur de la plaquette de guidage, au moins une première gorge de guidage d'un tuyau sensiblement en forme de U et une seconde gorge de guidage sensiblement rectiligne, suivant la direction longitudinale du bracelet, et agencée tangentiellement à la première gorge de guidage;
c) effectuer un moulage par injection au moyen du moule et du ou des tiroirs pour former le dispositif de guidage.

On peut également prévoir que le moule est agencé pour permettre le moulage simultané de l'organe de fermeture 14.

La description qui précède s'attache à décrire un mode de réalisation particulier à titre d'illustration non limitative et, l'invention n'est pas limitée à la mise en oeuvre de certaines caractéristiques particulières qui viennent d'être décrites, comme par exemple les formes spécifiquement illustrées et décrites pour le bracelet, son organe de fermeture ou la plaquette de guidage.

L'homme du métier ne rencontrera pas de difficulté particulière pour adapter le contenu de la présente divulgation à ses propres besoins et mettre en oeuvre un dispositif de guidage de tuyau(x) ne répondant qu'en partie aux caractéristiques qui viennent d'être présentées, sans sortir du cadre de la présente invention. A titre d'exemple, l'homme du métier pourra prévoir que des fentes sont ménagées dans l'épaisseur du bracelet pour permettre à la peau du patient de respirer, sans sortir du cadre de l'invention. De manière similaire, on pourra également prévoir qu'au lieu d'être cylindriques, les trous 15 sont évasés du côté par lequel le bouton 14 doit y être inséré, pour faciliter cette insertion sans pénaliser la qualité du maintien du bouton une fois qu'il est logé dans un trou donné.

A l'évidence, l'homme du métier pourra avoir avantage à réaliser le dispositif selon la présente invention en plusieurs tailles adaptées à des morphologies différentes de patients, comme par exemple au moins une taille enfant et au moins une taille adulte. En outre, il pourra également prévoir qu'un ou deux passants sont agencés sur le bracelet pour en retenir l'extrémité libre lorsqu'il est fermé. Ces passants peuvent être mobiles ou fixes et peuvent éventuellement être réalisés directement lors du moulage du bracelet.

## Revendications

1. Dispositif de guidage de tuyau(x) de perfusion (10), comportant un bracelet (11) muni d'un organe de fermeture (14) pour permettre son attache à un membre d'un patient et portant une plaquette de guidage (12) d'au moins un tuyau de perfusion (5, 6),
**caractérisé en ce que** ladite plaquette de guidage (12) présente, ménagées dans son épaisseur, au moins une première gorge de guidage (16) d'un tuyau (5), sensiblement en forme de U, et une seconde gorge de guidage (17) sensiblement rectiligne, suivant la direction longitudinale dudit bracelet (11), et agencée tangentiellement à ladite première gorge de guidage (16), de telle manière qu'un tuyau (5) peut être disposé en partie dans une première desdites gorges de guidage et en partie dans l'autre gorge de guidage.

2. Dispositif (10) selon la revendication 1, **caractérisé en ce que** ladite plaquette de guidage (12) comporte au moins une troisième gorge de guidage (18) sensiblement parallèle à ladite seconde gorge de guidage (17).

3. Dispositif (10) selon la revendication 1 ou 2, **caractérisé en ce que** chacune desdites gorges de guidage (16, 17, 18) présente, en section transversale à partir de la surface libre de ladite plaquette de guidage (12), deux parois (20 à 25) sensiblement parallèles se rejoignant en une courbe sensiblement en forme de demi-cercle, et **en ce que** chacune desdites gorges comprend au moins une région présentant au moins une surépaisseur (26) ménagée sur au moins l'une desdites parois, à proximité de la surface libre de ladite plaquette de guidage, et définissant un étranglement.

4. Dispositif (10) selon l'une quelconque des revendications 1 à 3, **caractérisé en ce que** ledit bracelet (11) et ladite plaquette de guidage (12) sont formés d'une pièce.

5. Dispositif (10) selon l'une quelconque des revendications précédentes, **caractérisé en ce que** ledit organe de fermeture comprend un bouton (14) agencé pour coopérer avec un trou (15) parmi une pluralité de trous ménagés dans ledit bracelet (11).

6. Dispositif (10) selon les revendications 4 et 5, **caractérisé en ce que** ledit bouton (14) est formé d'une pièce avec ledit bracelet (11) et ladite plaquette (12).

7. Dispositif (10) selon l'une quelconque des revendications précédentes, **caractérisé en ce que** ledit bracelet (11) présente une pluralité de fentes dans son épaisseur pour permettre à la peau du patient de respirer.

8. Procédé de fabrication d'un dispositif de guidage de tuyau(x) de perfusion (10), comportant un bracelet (11) muni d'un organe de fermeture (14) pour permettre son attache à un membre d'un patient et portant une plaquette de guidage (12) d'au moins un tuyau de perfusion (5, 6), **caractérisé en ce qu'**il comporte les étapes consistant à:
a) se munir d'un moule d'injection correspondant à la forme souhaitée pour ledit bracelet (11) et ladite plaquette de guidage (12);
b) se munir d'au moins un tiroir permettant de ménager, dans l'épaisseur de ladite plaquette de guidage, au moins une première gorge de guidage (16) d'un tuyau sensiblement en forme de U et une seconde gorge de guidage (17) sensiblement rectiligne, suivant la direction longitudinale dudit bracelet, et agencée tangentiellement à ladite première gorge de guidage;
c) effectuer un moulage par injection au moyen dudit moule et du ou desdits tiroirs pour former le dispositif de guidage.

9. Procédé selon la revendication 8, **caractérisé en ce que** ledit moule est agencé pour permettre également le moulage simultané d'une troisième gorge de guidage (18) d'un tuyau.

10. Procédé selon la revendication 8 ou 9, **caractérisé en ce que** ledit moule est agencé pour permettre également le moulage simultané dudit organe de fermeture.
